# EUROPEAN PATENT APPLICATION

(11) **EP 4 321 045 A1**
(43) Date of publication of application: **14.02.2024**
(21) Application number: 21948267.6
(22) Date of filing: 28.06.2021
(51) Int. Cl.: A24F 40/46

(54) **AEROSOL GENERATION SYSTEM**

(71) Applicant: Japan Tobacco, Inc., Tokyo, 105-6927 (JP)
(72) Inventor: YAMADA, Manabu, Tokyo 130-8603 (JP); INOUE, Yasunobu, Tokyo 130-8603 (JP)
(74) Representative: Hoffmann Eitle
(86) International application number: PCT/JP2021/024413
(87) International publication number: WO 2023/275955

(57) **Abstract**

Provided is an aerosol generation system capable of controlling the temperature distribution in a substrate to be heated. This aerosol generation system comprises: an elongated heat generation unit that, when energized, generates heat to heat an aerosol generating substrate from the inside thereof; and a pair of metal plates disposed to respectively cover the facing surfaces of the heat generation unit along the elongated shape. The heat generation unit includes at least two regions having different heat quantities.

## Description

### Technical Field

The present invention relates to aerosol generation systems.

### Background Art

Inhaler devices including electronic cigarettes and nebulizers that generate material to be inhaled by users are becoming widely popular. Such an inhaler device uses an aerosol source for generating an aerosol and a flavor source for imparting a flavor component to the generated aerosol, so as to be capable of generating a flavor-component-imparted aerosol. A user can taste the flavor by inhaling the flavor-component-imparted aerosol generated by the inhaler device.

In recent years, technology related to an inhaler device of a type that uses a stick-shaped substrate as an aerosol source or a flavor source is being actively developed. For example, Patent Literature 1 indicated below discloses a blade-shaped heater that is inserted into the stick-shaped substrate to heat the substrate from the inside thereof.

### Citation List

### Patent Literature

Patent Literature 1: CN 209807157 U

### Summary of Invention

### Technical Problem

However, with regard to the heater disclosed in Patent Literature 1 indicated above, the entire heater generates heat substantially uniformly, thus making it difficult to control the temperature distribution in the substrate to be heated. Therefore, in an inhaler device equipped with the heater disclosed in Patent Literature 1 indicated above, it is difficult to finely control the aerosol or flavor to be generated from the substrate by controlling the temperature distribution of the substrate.

The present invention has been made in view of the above problem, and an object of the present invention is to provide a new and improved aerosol generation system that can control the temperature distribution of the substrate to be heated. Solution to Problem

In order to solve the above problem, an aspect of the present invention provides an aerosol generation system including: a heat generator having a long shape, the heat generator generating heat by being supplied with electricity so as to heat an aerosol generating substrate from an inside thereof; and a pair of metal plates provided to respectively cover opposing surfaces of the heat generator along the long shape. The heat generator includes at least two regions where different amounts of heat are generated.

The aerosol generation system may further include the aerosol generating substrate into which the heat generator covered by the pair of metal plates is inserted.

A length of each of the pair of metal plates in a longitudinal direction of the long shape may be greater than a length of the heat generator

At a base side opposite a leading end to be inserted into the aerosol generating substrate, the pair of metal plates may be provided to extend further in the longitudinal direction relative to the heat generator

The heat generator may be supplied with the electricity between the pair of metal plates.

An insulator that limits supply of the electricity from the pair of metal plates to the heat generator may be further provided between the heat generator and at least one of the pair of metal plates.

The heat generator may include a plurality of heat generating elements. The plurality of heat generating elements may be provided separately from each other respectively in the regions where the different amounts of heat are generated.

The plurality of heat generating elements may have sizes, shapes, or properties different from each other.

The plurality of heat generating elements may be arranged separately from each other in a longitudinal direction of the long shape.

The heat generator at a leading end to be inserted into the aerosol generating substrate may have an angularly protruding shape at the leading end.

At least one of the pair of metal plates may further include a leading-end rib formed by bending an edge along the shape at the leading end of the heat generator

At least one of the pair of metal plates may include a rib formed by bending at least one of edges, in a lateral direction of the long shape, along the heat generator

The rib may be provided at each of opposite sides in the lateral direction of at least one of the pair of metal plates.

The heat generator may have a tabular shape. A thickness of the tabular shape may be smaller than 1/4 of a width of the tabular shape.

The pair of metal plates may be provided at opposite principal surfaces of the tabular shape of the heat generator.

The heat generator and the pair of metal plates may be adhered together by using a conductive adhesive paste.

The pair of metal plates may be composed of a nickel-containing iron alloy.

The heat generator may be a PTC heater.

The PTC heater may include barium titanate.

A temperature of the heat generated by the heat generator may be below 350°C.

### Advantageous Effects of Invention

As described above, the present invention can provide an aerosol generation system that can control the temperature distribution of the substrate to be heated.

### Brief Description of Drawings

[FIG. 1] FIG. 1 is a schematic diagram schematically illustrating a configuration example of an inhaler device.
[FIG. 2] FIG. 2 is a perspective view of a heater according to an embodiment of the present invention.
[FIG. 3] FIG. 3 is an exploded perspective view of a heater body included in the heater illustrated in FIG. 2.
[FIG. 4] FIG. 4 is a plan view illustrating an example of a detailed configuration of a heat generator
[FIG. 5] FIG. 5 is a plan view illustrating another example of the detailed configuration of the heat generator
[FIG. 6] FIG. 6 is a top view of a securing section included in the heater illustrated in FIG. 2.
[FIG. 7] FIG. 7 is an exploded perspective view of a heater body according to a first modification.
[FIG. 8] FIG. 8 is a top view of the heater body illustrated in FIG. 7.
[FIG. 9] FIG. 9 is an exploded perspective view of a heater body according to a second modification.
[FIG. 10] FIG. 10 is a top view of the heater body illustrated in FIG. 9.
[FIG. 11] FIG. 11 is an exploded perspective view of a heater body according to a third modification.
[FIG. 12] FIG. 12 is an exploded perspective view of a heater body according to a fourth modification.

### Description of Embodiments

A preferred embodiment of the present invention will be described in detail below with reference to the appended drawings. In this description and the drawings, structural elements having substantially identical functional configurations will be given the same reference signs, and redundant descriptions thereof will be omitted.

### 1. Configuration example of inhaler device

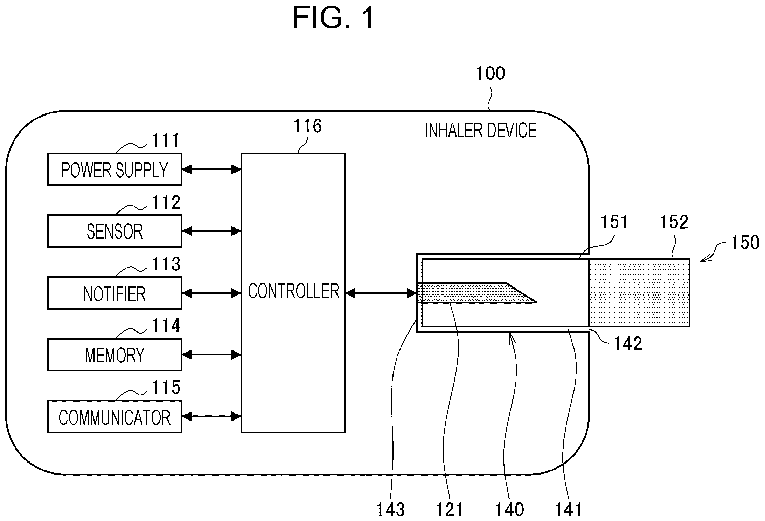

An inhaler device according to this configuration example generates an aerosol by heating a substrate containing an aerosol source from inside the substrate. A present configuration example will be described below with reference to FIG. 1.

FIG. 1 is a schematic diagram schematically illustrating a configuration example of the inhaler device. As illustrated in FIG. 1, an inhaler device 100 according to this configuration example includes a power supply 111, a sensor 112, a notifier 113, a memory 114, a communicator 115, a controller 116, a heater 121, and a container 140. Inhalation is performed by a user in a state where a stick substrate 150 is accommodated in the container 140. Each structural element will be sequentially described below.

The power supply 111 stores electric power. The power supply 111 supplies the electric power to the structural elements of the inhaler device 100. For example, the power supply 111 may be a rechargeable battery, such as a lithium ion secondary battery. The power supply 111 may be recharged by being connected to an external power supply by, for example, a USB (universal serial bus) cable. Alternatively, the power supply 111 may be recharged in a non-connected state with a power-transmitting device by wireless power transmission technology. As another alternative, the power supply 111 may be removable from the inhaler device 100 so as to be replaceable with a new power supply 111.

The sensor 112 detects various types of information regarding the inhaler device 100, and outputs the detected information to the controller 116. In an example, the sensor 112 may be a pressure sensor such as a microphone condenser, a flow sensor, or a temperature sensor. In such a case, when detecting a numerical value generated in accordance with the user's inhalation, the sensor 112 can output information indicating that the inhalation has been performed by the user to the controller 116. In another example, the sensor 112 is an input device, such as a button or a switch, receiving information input by the user. In particular, the sensor 112 may include a command button for starting/stopping aerosol generation. In such a case, the sensor 112 can output the information input by the user to the controller 116. In another example, the sensor 112 is a temperature sensor that detects the temperature of the heater 121. For example, the temperature sensor detects the temperature of the heater 121 based on an electrical resistance value of the heater 121. In such a case, the sensor 112 can detect the temperature of the stick substrate 150 accommodated in the container 140 based on the temperature of the heater 121.

The notifier 113 notifies the user of information. In an example, the notifier 113 is a light-emitting device, such as an LED (light-emitting diode). Accordingly, when the power supply 111 needs to be recharged, when the power supply 111 is being recharged, or when an abnormality has occurred in the inhaler device 100, the notifier 113 can emit light in different patterns of light, respectively. Each pattern of light is a concept involving colors and on/off timings. Together with or in place of the light-emitting device, the notifier 113 may be, for example, a display device that displays an image, a sound output device that outputs sound, and a vibration device that vibrates. The notifier 113 may also provide notification information indicating that inhalation by the user is possible. The notification information indicating that inhalation by the user is possible may be provided when the temperature of the stick substrate 150 heated by the heater 121 reaches a predetermined temperature.

The memory 114 stores various types of information for operation of the inhaler device 100. The memory 114 is, for example, a non-volatile storage medium, such as a flash memory. An example of the information stored in the memory 114 is information regarding the OS (operating system) of the inhaler device 100, such as the control contents of the various types of structural elements controlled by the controller 116. Another example of the information stored in the memory 114 is information regarding inhalation by the user, such as the number of times of inhalation, the inhalation time, and the accumulated inhalation time period.

The communicator 115 is a communication interface for exchanging information between the inhaler device 100 and another device. The communicator 115 performs communication in conformity with any wired or wireless communication standard. Such a communication standard may be, for example, a wireless LAN (local area network), a wired LAN, Wi-Fi (registered trademark), or Bluetooth (registered trademark). In an example, the communicator 115 transmits the information regarding the inhalation by the user to a smartphone to cause the smartphone to display the information regarding the inhalation by the user. In another example, the communicator 115 receives information about a new OS from a server to update the information about the OS stored in the memory 114.

The controller 116 functions as an arithmetic processing unit and a control device, and controls the overall operation in the inhaler device 100 in accordance with various programs. For example, the controller 116 is implemented by an electronic circuit, such as a CPU (central processing unit) or a microprocessor. Furthermore, the controller 116 may include a ROM (read only memory) that stores a program and arithmetic parameter to be used, and a RAM (random access memory) that temporarily stores an appropriately changing parameter. The inhaler device 100 executes various processes based on control by the controller 116. Examples of the processes controlled by the controller 116 include supplying of electric power from the power supply 111 to the other structural elements, recharging of the power supply 111, detection of information by the sensor 112, notification of information by the notifier 113, storing and reading of information by the memory 114, and exchanging of information by the communicator 115. Other processes executed by the inhaler device 100, such as input of information to each structural element and a process based on information output from each structural element, are also controlled by the controller 116.

The container 140 has an internal space 141 and holds the stick substrate 150 while accommodating a portion of the stick substrate 150 within the internal space 141. The container 140 has an opening 142 through which the internal space 141 communicates with the outside, and holds the stick substrate 150 inserted in the internal space 141 through the opening 142. For example, the container 140 is a tubular body having the opening 142 and a bottom 143 as a bottom surface, and defines the internal space 141 that is pillar-shaped. The container 140 has an inside diameter smaller than an outside diameter of the stick substrate 150 in at least a portion of the tubular body in the height direction, and may hold the stick substrate 150 while applying pressure around the stick substrate 150 inserted in the internal space 141. The container 140 also has a function for defining a flow path for air traveling through the stick substrate 150. An air inlet serving as an inlet for the air entering the flow path is disposed in, for example, the bottom 143. On the other hand, an air outlet serving as an outlet for the air exiting from the flow path is the opening 142.

The stick substrate 150 is a stick-shaped aerosol generating substrate. The stick substrate 150 includes a substrate 151 and an inhalation port 152.

The substrate 151 contains an aerosol source. The aerosol source atomizes by being heated, so that an aerosol is generated. The aerosol source may include, for example, a material derived from tobacco, such as a product obtained by forming shredded tobacco or tobacco raw material into a granular form, a sheet form, or a powder form. The aerosol source may also include a material not derived from tobacco and made from a plant (such as mint or herb) other than tobacco. If the inhaler device 100 is a medical inhaler, the aerosol source may include a medicine to be inhaled by a patient. The aerosol source is not limited to a solid and may be a liquid, such as polyhydric alcohol including glycerine and propylene glycol, or water. At least a portion of the substrate 151 is accommodated in the internal space 141 of the container 140 in the state where the stick substrate 150 is held by the container 140.

The inhalation port 152 is a member to be held in the user's mouth during inhalation. At least a portion of the inhalation port 152 protrudes from the opening 142 in the state where the stick substrate 150 is held by the container 140. When the user holds the inhalation port 152 protruding from the opening 142 in the user's mouth and inhales, air flows into the container 140 through the air inlet (not illustrated). The air flowing in travels through the internal space 141 of the container 140, that is, through the substrate 151, and reaches the inside of the user's mouth together with the aerosol generated from the substrate 151.

The heater 121 heats the aerosol source so as to atomize the aerosol source and generate the aerosol. As will be described in detail later, for example, the heater 121 is blade-shaped and is disposed to protrude from the bottom 143 of the container 140 to the internal space 141 of the container 140. Therefore, when the stick substrate 150 is inserted into the container 140, the blade-shaped heater 121 is inserted into the stick substrate 150 to pierce the substrate 151 of the stick substrate 150. When the heater 121 produces heat, the aerosol source contained in the stick substrate 150 atomizes by being heated from inside the stick substrate 150, whereby the aerosol is generated. The heater 121 produces heat when supplied with electric power from the power supply 111. In an example, when the sensor 112 detects that a predetermined user input has been performed, the aerosol may be generated by the heater 121 supplied with the electric power. When the temperature of the stick substrate 150 heated by the heater 121 reaches the predetermined temperature, inhalation by the user becomes possible. Subsequently, when the sensor 112 detects that a predetermined user input has been performed, the supply of electric power to the heater 121 may be stopped. In another example, in a time period in which the sensor 112 detects that the inhalation has been performed by the user, the aerosol may be generated by the heater 121 supplied with the electric power

The inhaler device 100 and the stick substrate 150 work in cooperation with each other to generate the aerosol to be inhaled by the user. Therefore, the combination of the inhaler device 100 and the stick substrate 150 may be regarded as an aerosol generation system.

### 2. Detailed configuration of heater

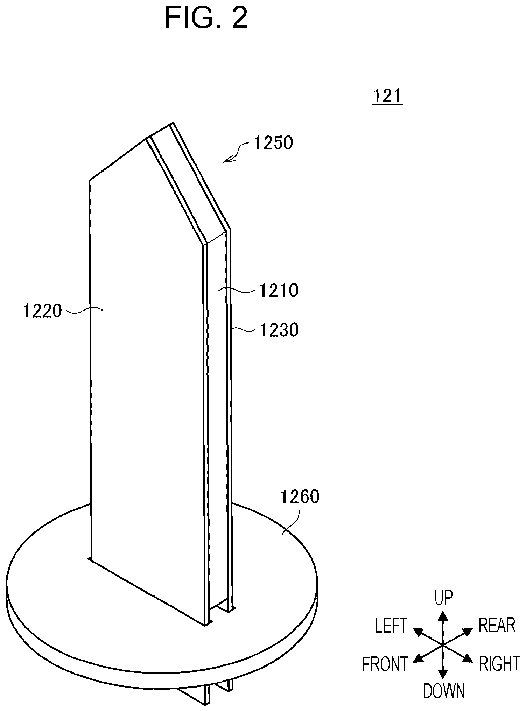

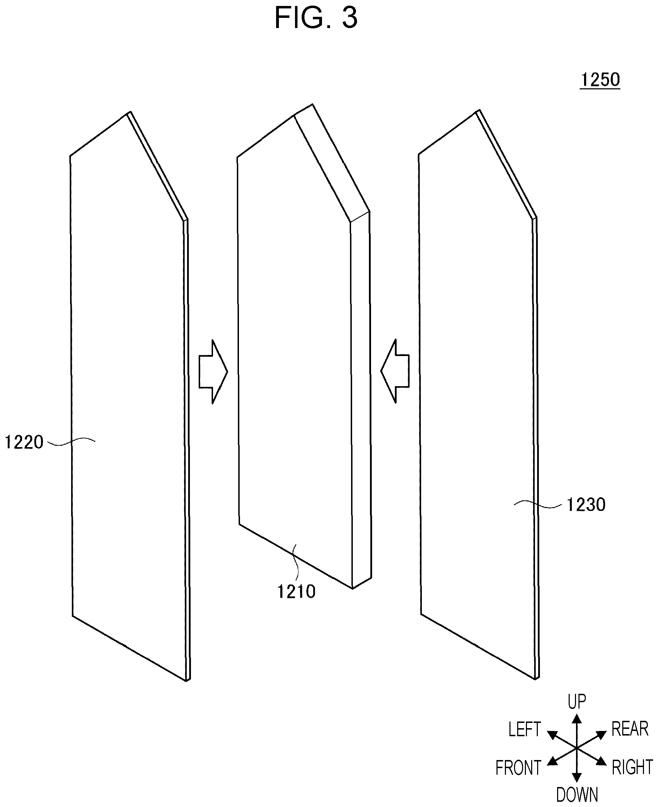

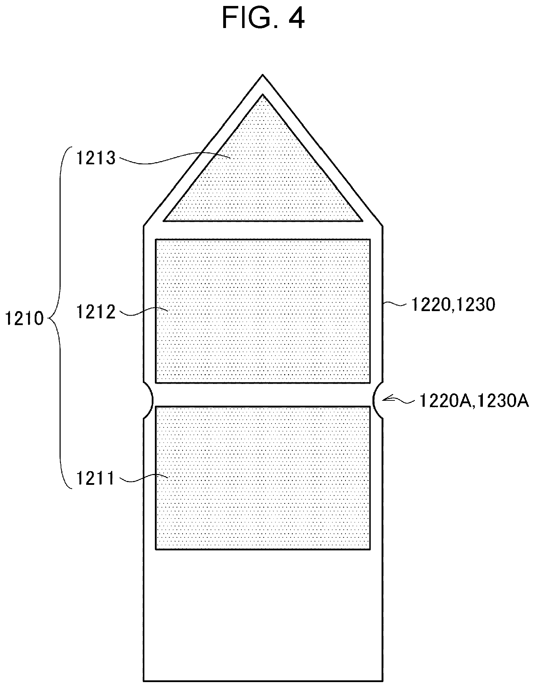

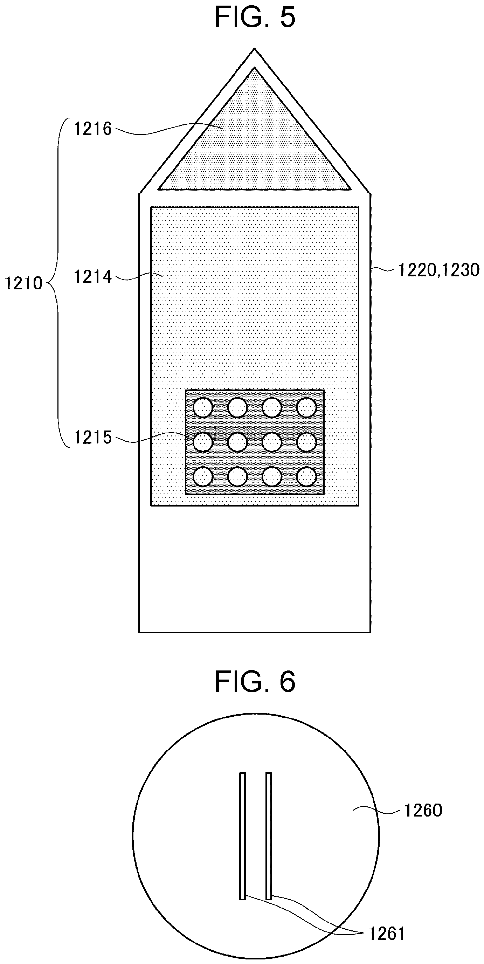

Next, the heater 121 included in the inhaler device 100 according to this embodiment will be described in further detail with reference to FIG. 2 to FIG. 6. FIG. 2 is a perspective view of the heater 121 according to this embodiment. FIG. 3 is an exploded perspective view of a heater body 1250 included in the heater 121 illustrated in FIG. 2. FIG. 4 is a plan view illustrating an example of a detailed configuration of a heat generator 1210. FIG. 5 is a plan view illustrating another example of the detailed configuration of the heat generator 1210. FIG. 6 is a top view of a securing section 1260 included in the heater 121 illustrated in FIG. 2.

As illustrated in FIG. 2, the heater 121 includes the heater body 1250 and the securing section 1260. The heater body 1250 is held at the securing section 1260 and is secured to, for example, a housing of the inhaler device 100 with the securing section 1260 interposed therebetween.

As illustrated in FIG. 3, the heater body 1250 includes the heat generator 1210, a first metal plate 1220, and a second metal plate 1230. The heater body 1250 can heat the stick substrate 150 from the inside thereof by using heat generated by the heat generator 1210 supplied with electricity via the first metal plate 1220 and the second metal plate 1230.

In FIG. 2 and FIG. 3, a direction in which the leading end of the heater body 1250 is inserted into the stick substrate 150 may also be referred to as "up direction", and a direction opposite the up direction may also be referred to as "down direction". A direction in which the first metal plate 1220, the heat generator 1210, and the second metal plate 1230 are bonded together may also be referred to as "front-rear direction", and a direction orthogonal to the up-down direction and the front-rear direction may also be referred to as "left-right direction".

The heat generator 1210 is a long-shaped member that generates heat by resistance heating. In detail, the heat generator 1210 may be a PTC (positive temperature coefficient) heater that generates heat when electricity is supplied between the first metal plate 1220 and the second metal plate 1230.

A PTC heater uses a resistor having properties (PTC properties) in which an electrical resistance value increases significantly when the temperature reaches a predetermined temperature (referred to as "Curie temperature") such that an electric current does not flow therethrough. By utilizing the PTC properties, a PTC heater can control the amount of supplied electricity without having to use a control device, so as to be capable of controlling the heating temperature below the Curie temperature. Therefore, a PTC heater can heat a target below the Curie temperature. For example, the heat generator 1210 may be a PTC heater with barium titanate (BaTiO₃) having the PTC properties as the resistor. In such a case, the heat generator 1210 can set the Curie temperature of the barium titanate to 350°C, so as to be capable of heating the stick substrate 150 to a temperature below 350°C.

In the heater 121 according to this embodiment, the heat generator 1210 includes at least two regions where different amounts of heat are generated. For example, the at least two regions where different amounts of heat are generated are arranged in the longitudinal direction of the long shape of the heat generator 1210.

Accordingly, the heat generator 1210 can heat the stick substrate 150 while controlling the temperature distribution of the heat generator 1210 in the longitudinal direction, so that the aerosol or the flavor to be generated from the stick substrate 150 can be controlled more finely. For example, in the heat generator 1210, the region located near the mouth of the user inhaling the aerosol (i.e., toward the leading end of the heater 121 to be inserted into the stick substrate 150) generates a smaller amount of heat, so that the temperature of the aerosol to be inhaled by the user can be reduced. Accordingly, the inhaler device 100 can prevent a high-temperature aerosol from being inhaled by the user, thereby providing a more comfortable flavor experience to the user

In detail, in order to have a plurality of regions where different amounts of heat are generated, the heat generator 1210 may include a plurality of heat generating elements (e.g., PTC heaters) that generate different amounts of heat.

For example, as illustrated in FIG. 4, the heat generator 1210 may include a first heat generating element 1211, a second heat generating element 1212, and a third heat generating element 1213 that are arranged in the up-down direction. The first heat generating element 1211, the second heat generating element 1212, and the third heat generating element 1213 are PTC heaters that generate different amounts of heat by being different from one another in terms of at least one of size, shape, and properties. The first heat generating element 1211, the second heat generating element 1212, and the third heat generating element 1213 are arranged separately from one another in the longitudinal direction of the long shape of the heat generator 1210.

Accordingly, the heat generator 1210 can supply electricity to the first heat generating element 1211, the second heat generating element 1212, and the third heat generating element 1213 in a parallel fashion via the first metal plate 1220 and the second metal plate 1230. Therefore, the heat generator 1210 can generate different amounts of heat in the regions where the first heat generating element 1211, the second heat generating element 1212, and the third heat generating element 1213 are provided.

In the case where the heat generator 1210 includes the plurality of heat generating elements that are separated from one another, the heat generator 1210 can also prevent the heat generating elements from cracking or prevent the heat generating elements from delaminating from the first metal plate 1220 and the second metal plate 1230 as a result of expansion or contraction of the heat generating elements.

In the case where the heat generator 1210 includes the plurality of heat generating elements, the first metal plate 1220 and the second metal plate 1230 that sandwich the heat generator 1210 therebetween may be provided with marks that indicate the positions of the plurality of heat generating elements. For example, in the case where the heat generator 1210 includes the first heat generating element 1211, the second heat generating element 1212, and the third heat generating element 1213, the first metal plate 1220 and the second metal plate 1230 that sandwich the heat generator 1210 therebetween may be provided with marks 1220A and 1230A, respectively.

The marks 1220A and 1230A are, for example, cutouts provided at the left and right side surfaces of the first metal plate 1220 and the second metal plate 1230. The marks 1220A and 1230A are provided for indicating the positions of the first heat generating element 1211, the second heat generating element 1212, and the third heat generating element 1213 that are separated from one another. With the marks 1220A and 1230A provided, the positions of the first heat generating element 1211, the second heat generating element 1212, and the third heat generating element 1213 can be controlled more precisely during manufacture of the heater 121. Consequently, the heater 121 can heat the stick substrate 150 with a more-precisely-controlled temperature distribution.

Alternatively, as illustrated in FIG. 5, the heat generator 1210 may include a heat generating element 1214 and a non-heat-generating element 1216. The heat generating element 1214 is a PTC heater, and the non-heat-generating element 1216 is an organic or inorganic insulator. The heat generating element 1214 and the non-heat-generating element 1216 are arranged separately from each other in the longitudinal direction of the long shape of the heat generator 1210.

Accordingly, in the heat generator 1210, the region where the heat generating element 1214 is disposed can serve as a region where heat is generated, and the region where the non-heat-generating element 1214 is disposed can serve as a region where heat is not generated. For example, in the heat generator 1210, the region located toward the leading end of the heater 121 to be inserted into the stick substrate 150 may serve as the region where the non-heat-generating element 1216 is disposed and where heat is not generated, and the region located toward the trailing end opposite the leading end may serve as the region where the heat generating element 1214 is disposed and where heat is generated.

The heat generating element 1214 of the heat generator 1210 may be provided with a region where a mask member 1215 is bonded. The mask member 1215 is an organic or inorganic insulator having patterned openings. The mask member 1215 is provided for controlling the amount of electricity supplied to the heat generating element 1214 from the first metal plate 1220 and the second metal plate 1230 that sandwich the heat generator 1210 therebetween. The mask member 1215 may be composed of any of a liquid-crystal polymer, an insulative ceramic material, silicone resin, and various types of glass.

In detail, in the region where the mask member 1215 is bonded, the supply of electricity to the heat generating element 1214 from the first metal plate 1220 and the second metal plate 1230 is limited to the region of the openings provided in the mask member 1215. Therefore, by bonding the mask member 1215 to the heat generating element 1214, the heat generator 1210 can reduce the amount of electricity supplied to the region where the mask member 1215 is bonded, so that the amount of heat generated in the region can be reduced. Accordingly, the heat generator 1210 can vary the amount of heat generated for each region without including a plurality of heat generating elements 1214. The pattern of the openings provided in the mask member 1215 is arbitrary and is not particularly limited.

Furthermore, the heat generating element 1214 of the heat generator 1210 may be provided with a region where a resistor that induces resistance heating is bonded. In the region where the resistor is bonded, if the same amount of electricity is supplied from the first metal plate 1220 and the second metal plate 1230, the amount of heat generated in the region due to the resistance heating from the resistor increases. Accordingly, by bonding the resistor to the heat generating element 1214, the heat generator 1210 can increase the amount of heat generated in the region where the resistor is bonded. Therefore, the heat generator 1210 can vary the amount of heat generated for each region without including a plurality of heat generating elements 1214.

The heat generator 1210 may have a long tabular shape extending in the up-down direction as its overall outer shape including the plurality of regions. Specifically, when the multiple regions where different amounts of heat are generated are connected to each other while neglecting gaps therebetween in the up-down direction, the heat generator 1210 may have a long tabular shape extending in the up-down direction. The longitudinal direction of the long shape of the heat generator 1210 corresponds to the up-down direction, whereas the lateral direction of the long shape corresponds to the left-right direction. By having a tabular shape, the heat generator 1210 has a rectangular cross-sectional shape that is orthogonal to the longitudinal direction (i.e., the up-down direction) of the long shape. Accordingly, as compared with a case where the heat generator 1210 has a circular cross-sectional shape, the cross-sectional shape can have a longer perimeter even with the same cross-sectional area. Therefore, the heat generator 1210 can allow for a larger contact area between the heater 121 and the stick substrate 150 to which the heater 121 is to be inserted, whereby the stick substrate 150 can be heated more efficiently. For example, the tabular shape of the heat generator 1210 may have a thickness smaller than 1/4 of the width of the long shape in the lateral direction (i.e., the left-right direction).

The heat generator 1210 at the leading end to be inserted into the stick substrate 150 may have an angularly protruding shape toward the leading end (i.e., in the up direction). The angular shape extending toward the leading end may have an acute angle, a right angle, or an obtuse angle. With regard to the heat generator 1210, the leading end (i.e., the upper end) thereof to be inserted into the stick substrate 150 has a pointy shape like a sword tip, so that the heater 121 can be inserted into the stick substrate 150 more readily.

The first metal plate 1220 and the second metal plate 1230 are a pair of electrode plates sandwiching the heat generator 1210 therebetween. In detail, the first metal plate 1220 and the second metal plate 1230 may be provided at opposite principal surfaces opposing each other in the front-rear direction of the tabular heat generator 1210. The first metal plate 1220 and the second metal plate 1230 are provided apart from each other to prevent a short-circuit.

The first metal plate 1220 and the second metal plate 1230 are bonded to the heat generator 1210 by using a conductive adhesive paste, so that electricity can be supplied to the heat generator 1210. An example of the conductive adhesive paste that can be used is a so-called anisotropic conductive adhesive having conductive particles uniformly distributed within an epoxy-based adhesive.

The first metal plate 1220 and the second metal plate 1230 may be composed of metal with a low thermal expansion coefficient. For example, the first metal plate 1220 and the second metal plate 1230 may be composed of a nickel (Ni) containing iron alloy with a low thermal expansion coefficient, such as Invar (registered trademark). Accordingly, delamination of the first metal plate 1220 and the second metal plate 1230 from the heat generator 1210 due to thermal expansion occurring when the heat generator 1210 generates heat can be suppressed.

The first metal plate 1220 and the second metal plate 1230 may be provided to cover the heat generator 1210 by having a shape that conforms with the shape of the heat generator 1210. In detail, the first metal plate 1220 and the second metal plate 1230 may each have a shape that extends further in the longitudinal direction (i.e., the up-down direction) relative to the long shape of the heat generator 1210. For example, the first metal plate 1220 and the second metal plate 1230 may each be similar to the heat generator 1210 in having a pentagonal tabular shape whose apex exists at the leading end (i.e., the upper end) to be inserted into the stick substrate 150 and that extends in the up-down direction. The first metal plate 1220 and the second metal plate 1230 may have the same shape or may have shapes different from each other.

Each of the first metal plate 1220 and the second metal plate 1230 at the trailing end (i.e., the lower end) opposite the leading end may extend further downward relative to the trailing end of the heat generator 1210. The first metal plate 1220 and the second metal plate 1230 in regions extending further downward relative to the trailing end of the heat generator 1210 may be provided with, for example, the securing section 1260.

The securing section 1260 is a structural member that secures the heater body 1250 to the housing of the inhaler device 100. In detail, the securing section 1260 is provided with an insertion section 1261 having a slit-like recess structure or a through-hole structure. The first metal plate 1220 and the second metal plate 1230 are inserted into the insertion section 1261, so that the securing section 1260 can hold the heater body 1250.

The securing section 1260 may be composed of an engineering plastic material. An engineering plastic material has high heat resistance and high mechanical strength and can be formed into a desired shape inexpensively by injection molding, and is therefore suitable as a material for forming a structural member. For example, the securing section 1260 may be composed of PEEK (polyether ether ketone), which is a type of engineering plastic material. PEEK is thermoplastic resin having extremely high heat resistance and also having high dimensional stability. Therefore, when the securing section 1260 is composed of PEEK, the securing section 1260 is less likely to be affected by the heat generated by the heat generator 1210.

For example, as illustrated in FIG. 6, the securing section 1260 may have a circular tabular shape. The securing section 1260 may be provided with the insertion section 1261 having two recesses or through-holes. The first metal plate 1220 and the second metal plate 1230 are respectively inserted into the two recesses or through-holes, so that the securing section 1260 can hold the heater body 1250. Alternatively, the securing section 1260 may be provided with the insertion section 1261 having a single recess or through-hole. The first metal plate 1220 and the second metal plate 1230 are collectively inserted into the single recess or through-hole, so that the securing section 1260 can hold the heater body 1250.

In the heater 121 included in the inhaler device 100 according to this embodiment, the securing section 1260 holds the first metal plate 1220 and the second metal plate 1230 instead of the heat generator 1210, thereby securing the heater body 1250 to the housing of the inhaler device 100. Accordingly, the securing section 1260 can further reduce the possibility of transmission of the heat generated from the heat generator 1210 to the housing of the inhaler device 100.

In the case where each of the first metal plate 1220 and the second metal plate 1230 at the trailing end (i.e., the lower end) opposite the leading end extends further downward relative to the trailing end of the heat generator 1210, the securing section 1260 may hold the first metal plate 1220 and the second metal plate 1230 at the regions extending further downward relative to the trailing end of the heat generator 1210. By holding the heater body 1250 at the regions located away from the heat generator 1210, the securing section 1260 is less likely to be affected by the heat generated by the heat generator 1210. Accordingly, with regard to the securing section 1260, the material thereof can be selected more flexibly in view of not only heat resistance but also machinability and cost. For example, the material that can be used for forming the securing section 1260 may be resin with a melting point or glass transition point lower than that of, for example, metal.

With the above configuration, the heater 121 according to this embodiment can heat the stick substrate 150 while controlling the temperature distribution of the heat generator 1210, so that the aerosol or the flavor to be generated from the stick substrate 150 can be controlled more finely. Therefore, the inhaler device 100 according to this embodiment can provide a more finely controlled flavor experience to the user

### 3. Modifications

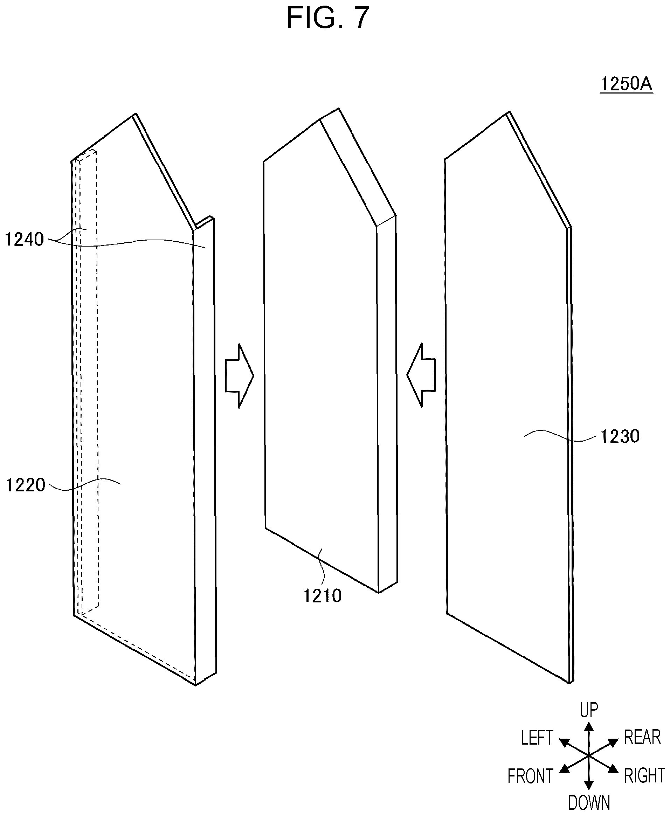

First to fourth modifications of the heater body 1250 according to this embodiment will now be described with reference to FIG. 7 to FIG. 12.

### (First Modification)

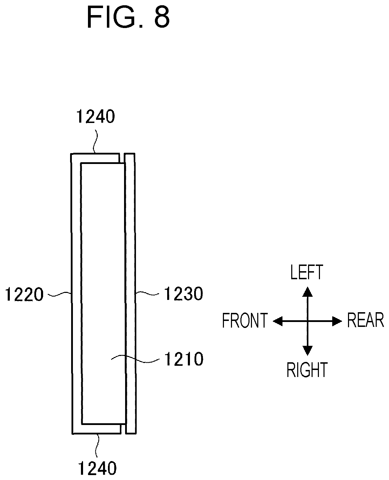

FIG. 7 is an exploded perspective view of a heater body 1250A according to a first modification. FIG. 8 is a top view of the heater body 1250A illustrated in FIG. 7.

In FIG. 7 and FIG. 8, the up-down direction, the front-rear direction, and the left-right direction are defined similarly to FIG. 2 and FIG. 3. In detail, a direction in which the leading end of the heater body 1250A is inserted into the stick substrate 150 may also be referred to as "up direction", and a direction opposite the up direction may also be referred to as "down direction". A direction in which the first metal plate 1220, the heat generator 1210, and the second metal plate 1230 are bonded together may also be referred to as "front-rear direction", and a direction orthogonal to the up-down direction and the front-rear direction may also be referred to as "left-right direction".

As illustrated in FIG. 7 and FIG. 8, in the heater body 1250A according to the first modification, at least one of the first metal plate 1220 and the second metal plate 1230 is provided with ribs 1240. Since the heat generator 1210 is as described with reference to FIG. 2 to FIG. 6, the description thereof will be omitted here.

In detail, the ribs 1240 are formed by bending opposite edges, in the lateral direction (i.e., the left-right direction) of the long shape of the first metal plate 1220, along the outer shape of the heat generator 1210. For example, if the first metal plate 1220 has a pentagonal shape extending in the up-down direction, the ribs 1240 may be formed by bending the opposite left and right edges extending from the first metal plate 1220.

With the ribs 1240 provided, the first metal plate 1220 has increased strength in the front-rear direction (i.e., the normal direction to the principal surfaces of the first metal plate 1220) in which the ribs 1240 are bent, so that deformation in the normal direction can be suppressed. Accordingly, the heater body 1250A is less likely to deform in the normal direction (i.e., the front-rear direction) to the principal surfaces of the first metal plate 1220, so that the possibility of breakage of the heater body 1250A in the normal direction can be reduced. With the heater body 1250A according to the first modification, the heater 121 can have increased strength in the front-rear direction that is relatively lower than the strength in the up-down direction and the left-right direction, so that the possibility of breakage of the heater 121 when inserted into the stick substrate 150 can be reduced.

### (Second Modification)

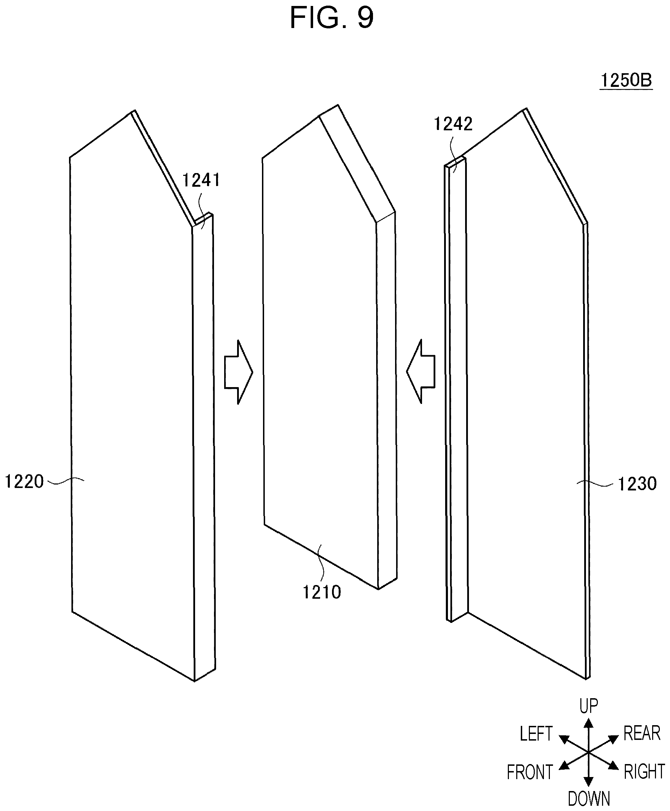

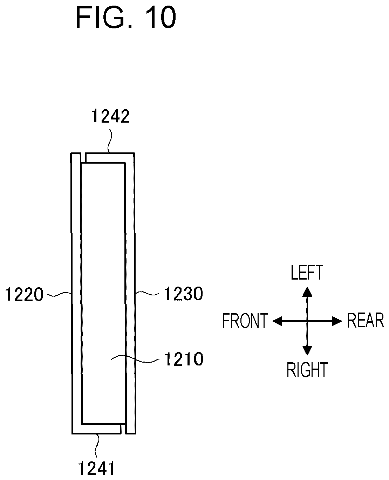

FIG. 9 is an exploded perspective view of a heater body 1250B according to a second modification. FIG. 10 is a top view of the heater body 1250B illustrated in FIG. 9.

In FIG. 9 and FIG. 10, the up-down direction, the front-rear direction, and the left-right direction are defined similarly to FIG. 2 and FIG. 3. In detail, a direction in which the leading end of the heater body 1250B is inserted into the stick substrate 150 may also be referred to as "up direction", and a direction opposite the up direction may also be referred to as "down direction". A direction in which the first metal plate 1220, the heat generator 1210, and the second metal plate 1230 are bonded together may also be referred to as "front-rear direction", and a direction orthogonal to the up-down direction and the front-rear direction may also be referred to as "left-right direction".

As illustrated in FIG. 7 and FIG. 8, in the heater body 1250B according to the second modification, the first metal plate 1220 is provided with a first rib 1241, and the second metal plate 1230 is provided with a second rib 1242. Since the heat generator 1210 is as described with reference to FIG. 2 to FIG. 6, the description thereof will be omitted here.

In detail, the first rib 1241 may be formed by bending one of the edges, in the lateral direction (i.e., the left-right direction) of the long shape of the first metal plate 1220, along the outer shape of the heat generator 1210. The second rib 1242 may be formed by bending the other one of the edges, in the lateral direction (i.e., the left-right direction) of the long shape of the second metal plate 1230, along the outer shape of the heat generator 1210. For example, if the first metal plate 1220 and the second metal plate 1230 have a pentagonal shape extending in the up-down direction, the first rib 1241 may be formed by bending the right edge extending from the first metal plate 1220. Moreover, the second rib 1242 may be formed by bending the left edge extending from the second metal plate 1230.

With the first rib 1241 and the second rib 1242 provided, the first metal plate 1220 and the second metal plate 1230 have increased strength in the front-rear direction in which the first rib 1241 and the second rib 1242 are bent, so that deformation in the normal direction can be suppressed. Accordingly, the heater body 1250B is less likely to deform in the normal direction (i.e., the front-rear direction) to the principal surfaces of the first metal plate 1220 and the second metal plate 1230, so that the possibility of breakage of the heater 121 in the normal direction can be reduced.

Specifically, the first rib 1241 and the second rib 1242 may be provided at both of the pair of electrode plates (i.e., the first metal plate 1220 and the second metal plate 1230). In such a case, the heater body 1250B according to the second modification is similar to the heater body 1250A provided with the ribs 1240 only at the first metal plate 1220 in that the possibility of breakage of the heater 121 when inserted into the stick substrate 150 can be reduced.

### (Third Modification)

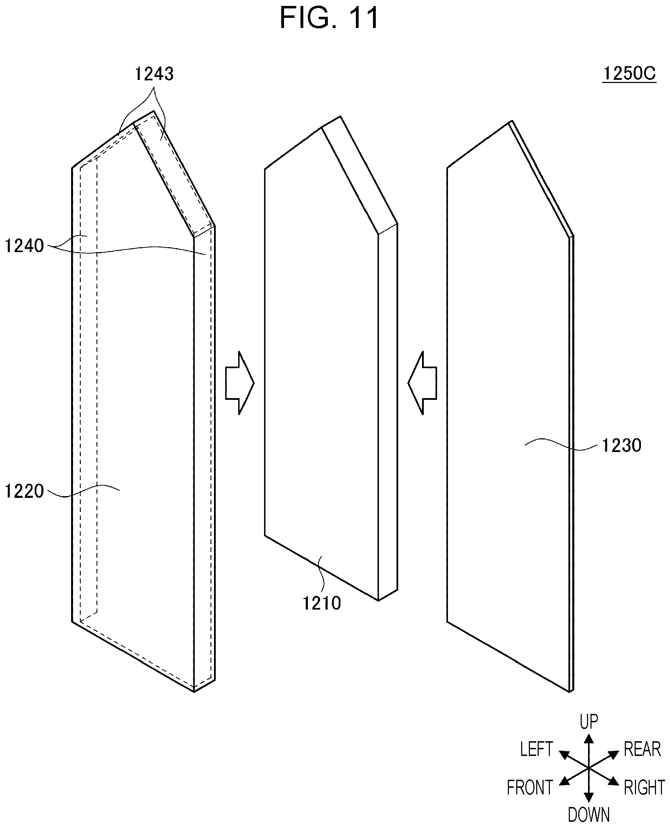

FIG. 11 is an exploded perspective view of a heater body 1250C according to a third modification.

In FIG. 11, the up-down direction, the front-rear direction, and the left-right direction are defined similarly to FIG. 2 and FIG. 3. In detail, a direction in which the leading end of the heater body 1250C is inserted into the stick substrate 150 may also be referred to as "up direction", and a direction opposite the up direction may also be referred to as "down direction". A direction in which the first metal plate 1220, the heat generator 1210, and the second metal plate 1230 are bonded together may also be referred to as "front-rear direction", and a direction orthogonal to the up-down direction and the front-rear direction may also be referred to as "left-right direction".

As illustrated in FIG. 11, in addition to the ribs 1240, the heater body 1250C according to the third modification is further provided with leading-end ribs 1243 in conformity with the angularly protruding shape toward the leading end (i.e., in the up direction) of the heat generator 1210. Since the heat generator 1210 is as described with reference to FIG. 2 to FIG. 6, the description thereof will be omitted here.

In detail, the leading-end ribs 1243 may be formed by bending upper edges (located toward the leading end of the heat generator 1210) of the first metal plate 1220 along the outer shape of the heat generator 1210. For example, if the first metal plate 1220 has a pentagonal shape extending in the up-down direction, the leading-end ribs 1243 may be formed by bending two upper edges of the first metal plate 1220. In such a case, the first metal plate 1220 has the ribs 1240 and the leading-end ribs 1243 at four edges excluding the lower edge of the pentagonal shape.

With the leading-end ribs 1243 provided, the first metal plate 1220 can cover the sword-tip-like pointy-shaped leading end (i.e., the upper end) of the heat generator 1210 with the leading-end ribs 1243. Accordingly, when the heater 121 is inserted into the stick substrate 150, the heater body 1250C can prevent delamination of the first metal plate 1220 and the second metal plate 1230 from the heat generator 1210 due to stress acting among the heat generator 1210, the first metal plate 1220, and the second metal plate 1230. Therefore, the heater body 1250C can further improve the durability of the heater 121 with respect to insertion thereof into the stick substrate 150.

### (Fourth Modification)

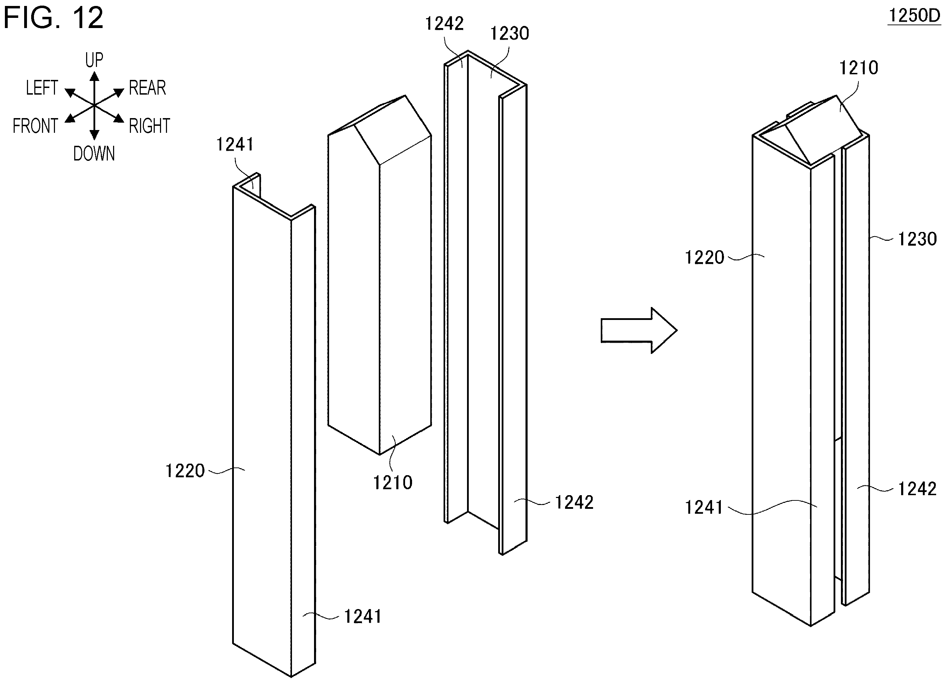

FIG. 12 is an exploded perspective view of a heater body 1250D according to a fourth modification.

In FIG. 12, the up-down direction, the front-rear direction, and the left-right direction are defined similarly to FIG. 2 and FIG. 3. In detail, a direction in which the leading end of the heater body 1250D is inserted into the stick substrate 150 may also be referred to as "up direction", and a direction opposite the up direction may also be referred to as "down direction". A direction in which the first metal plate 1220, the heat generator 1210, and the second metal plate 1230 are bonded together may also be referred to as "front-rear direction", and a direction orthogonal to the up-down direction and the front-rear direction may also be referred to as "left-right direction".

As illustrated in FIG. 12, in the heater body 1250D according to the fourth modification, the first metal plate 1220 is provided with first ribs 1241, and the second metal plate 1230 is provided with second ribs 1242.

In detail, the first ribs 1241 may be formed by bending opposite edges, in the lateral direction (i.e., the left-right direction) of the long shape of the first metal plate 1220, along the outer shape of the heat generator 1210. The second ribs 1242 may be formed by bending opposite edges, in the lateral direction (i.e., the left-right direction) of the long shape of the second metal plate 1230, along the outer shape of the heat generator 1210. For example, if the first metal plate 1220 and the second metal plate 1230 have a pentagonal shape extending in the up-down direction, the first ribs 1241 may be formed by bending the opposite edges in the lateral direction of the first metal plate 1220. Moreover, the second ribs 1242 may be formed by bending the opposite edges in the lateral direction of the second metal plate 1230.

In such a case, the heat generator 1210 may have a shape with a larger thickness to prevent a short-circuit between the first ribs 1241 and the second ribs 1242. For example, the heat generator 1210 may have a prismatic shape extending in the up-down direction. The heat generator 1210 at the leading end to be inserted into the stick substrate 150 may protrude to form a ridge toward the leading end (i.e., in the up direction).

With the first ribs 1241 and the second ribs 1242 provided, the first metal plate 1220 and the second metal plate 1230 have increased strength in the front-rear direction in which the first ribs 1241 and the second ribs 1242 are bent, so that deformation in the normal direction can be suppressed. Accordingly, the heater body 1250D is less likely to deform in the normal direction (i.e., the front-rear direction) to the principal surfaces of the first metal plate 1220 and the second metal plate 1230, so that the possibility of breakage of the heater body 1250D in the normal direction can be reduced. Consequently, the heater body 1250D according to the fourth modification is similar to the heater body 1250A according to the first modification in that the possibility of breakage of the heater 121 when inserted into the stick substrate 150 can be reduced.

Although a preferred embodiment of the present invention has been described in detail above with reference to the appended drawings, the present invention is not limited to this example. It is apparent to a person with a common knowledge of the technical field to which the present invention belongs that various modifications and alterations are conceivable within the scope of the technical ideas defined in the claims, and it is to be understood that such modifications and alterations naturally belong to the technical scope of the present invention.

The following configurations also belong to the technical scope of the present invention.
(1) An aerosol generation system comprising:
   a heat generator having a long shape, the heat generator generating heat by being supplied with electricity so as to heat an aerosol generating substrate from an inside thereof; and
   a pair of metal plates provided to respectively cover opposing surfaces of the heat generator along the long shape,
   wherein the heat generator includes at least two regions where different amounts of heat are generated.
(2) The aerosol generation system according to (1), further comprising the aerosol generating substrate into which the heat generator covered by the pair of metal plates is inserted.
(3) The aerosol generation system according to (1) or (2), wherein a length of each of the pair of metal plates in a longitudinal direction of the long shape is greater than a length of the heat generator.
(4) The aerosol generation system according to (3), wherein, at a base side opposite a leading end to be inserted into the aerosol generating substrate, the pair of metal plates are provided to extend further in the longitudinal direction relative to the heat generator.
(5) The aerosol generation system according to any one of (1) to (4), wherein the heat generator is supplied with the electricity between the pair of metal plates.
(6) The aerosol generation system according to (5), wherein an insulator that limits supply of the electricity from the pair of metal plates to the heat generator is further provided between the heat generator and at least one of the pair of metal plates.
(7) The aerosol generation system according to any one of (1) to (6),
   wherein the heat generator includes a plurality of heat generating elements, and
   wherein the plurality of heat generating elements are provided separately from each other respectively in the regions where the different amounts of heat are generated.
(8) The aerosol generation system according to (7), wherein the plurality of heat generating elements have sizes, shapes, or properties different from each other.
(9) The aerosol generation system according to (7) or (8), wherein the plurality of heat generating elements are arranged separately from each other in a longitudinal direction of the long shape.
(10) The aerosol generation system according to any one of (1) to (9), wherein the heat generator at a leading end to be inserted into the aerosol generating substrate has an angularly protruding shape at the leading end.
(11) The aerosol generation system according to (10), wherein at least one of the pair of metal plates further includes a leading-end rib formed by bending an edge along the shape at the leading end of the heat generator
(12) The aerosol generation system according to any one of (1) to (11), wherein at least one of the pair of metal plates includes a rib formed by bending at least one of edges, in a lateral direction of the long shape, along the heat generator
(13) The aerosol generation system according to (12), wherein the rib is provided at each of opposite sides in the lateral direction of at least one of the pair of metal plates.
(14) The aerosol generation system according to any one of (1) to (13),
   wherein the heat generator has a tabular shape, and
   wherein a thickness of the tabular shape is smaller than 1/4 of a width of the tabular shape.
(15) The aerosol generation system according to (14), wherein the pair of metal plates are provided at opposite principal surfaces of the tabular shape of the heat generator
(16) The aerosol generation system according to any one of (1) to (15), wherein the heat generator and the pair of metal plates are adhered together by using a conductive adhesive paste.
(17) The aerosol generation system according to any one of (1) to (16), wherein the pair of metal plates are composed of a nickel-containing iron alloy.
(18) The aerosol generation system according to any one of (1) to (17), wherein the heat generator is a PTC heater.
(19) The aerosol generation system according to (18), wherein the PTC heater includes barium titanate.
(20) The aerosol generation system according to (18) or (19), wherein a temperature of the heat generated by the heat generator is below 350°C.

### Reference Signs List

- 100: inhaler device
- 111: power supply
- 112: sensor
- 113: notifier
- 114: memory
- 115: communicator
- 116: controller
- 121: heater
- 140: container
- 141: internal space
- 142: opening
- 143: bottom
- 150: stick substrate
- 151: substrate
- 152: inhalation port
- 1210: heat generator
- 1211: first heat generating element
- 1212: second heat generating element
- 1213: third heat generating element
- 1214: heat generating element
- 1215: mask member
- 1216: non-heat-generating element
- 1220: first metal plate
- 1230: second metal plate
- 1220A, 1230A: mark
- 1240: rib
- 1241: first rib
- 1242: second rib
- 1243: leading-end rib
- 1250, 1250A, 1250B, 1250C, 1250D: heater body
- 1260: securing section
- 1261: insertion section

## Claims

1. An aerosol generation system comprising:
a heat generator having a long shape, the heat generator generating heat by being supplied with electricity so as to heat an aerosol generating substrate from an inside thereof; and
a pair of metal plates provided to respectively cover opposing surfaces of the heat generator along the long shape,
wherein the heat generator includes at least two regions where different amounts of heat are generated.

2. The aerosol generation system according to claim 1, further comprising the aerosol generating substrate into which the heat generator covered by the pair of metal plates is inserted.

3. The aerosol generation system according to claim 1 or 2, wherein a length of each of the pair of metal plates in a longitudinal direction of the long shape is greater than a length of the heat generator.

4. The aerosol generation system according to claim 3, wherein, at a base side opposite a leading end to be inserted into the aerosol generating substrate, the pair of metal plates are provided to extend further in the longitudinal direction relative to the heat generator.

5. The aerosol generation system according to any one of claims 1 to 4, wherein the heat generator is supplied with the electricity between the pair of metal plates.

6. The aerosol generation system according to claim 5, wherein an insulator that limits supply of the electricity from the pair of metal plates to the heat generator is further provided between the heat generator and at least one of the pair of metal plates.

7. The aerosol generation system according to any one of claims 1 to 6,
wherein the heat generator includes a plurality of heat generating elements, and
wherein the plurality of heat generating elements are provided separately from each other respectively in the regions where the different amounts of heat are generated.

8. The aerosol generation system according to claim 7, wherein the plurality of heat generating elements have sizes, shapes, or properties different from each other.

9. The aerosol generation system according to claim 7 or 8, wherein the plurality of heat generating elements are arranged separately from each other in a longitudinal direction of the long shape.

10. The aerosol generation system according to any one of claims 1 to 9, wherein the heat generator at a leading end to be inserted into the aerosol generating substrate has an angularly protruding shape at the leading end.

11. The aerosol generation system according to claim 10, wherein at least one of the pair of metal plates further includes a leading-end rib formed by bending an edge along the shape at the leading end of the heat generator.

12. The aerosol generation system according to any one of claims 1 to 11, wherein at least one of the pair of metal plates includes a rib formed by bending at least one of edges, in a lateral direction of the long shape, along the heat generator

13. The aerosol generation system according to claim 12, wherein the rib is provided at each of opposite sides in the lateral direction of at least one of the pair of metal plates.

14. The aerosol generation system according to any one of claims 1 to 13,
wherein the heat generator has a tabular shape, and
wherein a thickness of the tabular shape is smaller than 1/4 of a width of the tabular shape.

15. The aerosol generation system according to claim 14, wherein the pair of metal plates are provided at opposite principal surfaces of the tabular shape of the heat generator

16. The aerosol generation system according to any one of claims 1 to 15, wherein the heat generator and the pair of metal plates are adhered together by using a conductive adhesive paste.

17. The aerosol generation system according to any one of claims 1 to 16, wherein the pair of metal plates are composed of a nickel-containing iron alloy.

18. The aerosol generation system according to any one of claims 1 to 17, wherein the heat generator is a PTC heater.

19. The aerosol generation system according to claim 18, wherein the PTC heater includes barium titanate.

20. The aerosol generation system according to claim 18 or 19, wherein a temperature of the heat generated by the heat generator is below 350°C.
